# EUROPEAN PATENT SPECIFICATION

(11) **EP 0 780 055 B1**
(45) Date of publication and mention of the grant of the patent: **11.12.2002**
(21) Application number: 96202863.5
(22) Date of filing: 15.10.1996
(51) Int. Cl.: A23F 5/40, A23F 5/38

(54) **Nutritional coffee composition**
Nahrhafte Kaffeezusammensetzung
Composition de café nutritive

(30) Priority: 27.10.1995 US 550093
(43) Date of publication of application: 25.06.1997
(73) Proprietor: SOCIETE DES PRODUITS NESTLE S.A., 1800 Vevey (CH)
(72) Inventor: Sartorio, Claude, Lake Bluff, Il 60044 (US); Burri, Josef, 1066 Epalinges (CH); Chang, Shen-Youn, Wadsworth, Il 60083 (US); Lin, Paul, Fullerton, CA 92633 (US); Wehrspann, Olaf, 1350 Orbe (CH)

(56) References cited:
- EP-A- 0 052 919
- EP-A- 0 154 192
- EP-A- 0 458 310
- EP-A- 0 679 338
- FR-A- 2 258 799
- US-A- 3 458 319
- US-A- 3 706 572
- US-A- 5 433 962
- DATABASE WPI Week 8815 Derwent Publications Ltd., London, GB; AN 88-103895 XP002024027 & SU 1 335 243 A (A MED FOOD SUPPLY)

## Description

This invention relates to a nutritional composition in powder form which contains soluble coffee solids and to beverages produced from the nutritional composition. The invention further relates to processes for the production of the nutritional composition and to methods of providing patients with nutritional support.

Patent publication EP 0 154 192 describes a powdered beverage composition for use in preparing a "cappuccino" style. It comprises fats, proteins, lactose and other carbohydrates. The ratio of protein to lactose is between 1/3.5 and 1/5. Previously, patent number US 4,438,147 had described a procedure for the manufacture of a powdered beverage composition comprising non-dairy fats, a non-dairy carbohydrate and a foam stabiliser containing proteins such as sodium caseinate. These publications do not address nutritional aspects.

Patients suffering from a loss of nutrients require adequate nutritional support. Inadequate nutritional support can result in malnutrition associated complications, such as prolonged negative nitrogen balance and depletion of somatic and visceral protein levels. Thus, the goal of nutritional support is to maintain body mass, provide nitrogen and energy in adequate amounts to support healing, meet metabolic demands characterised by the degree of stress, and support immune function.

A traditional form of nutritional support is administering whole protein liquid feedings to the patient. Whole protein feedings are offered as a dietary supplement that typically can be consumed as partial or total meal replacements in hospitals, nursing homes and by home patients. Dietary supplements are generally useful for patients who are able to eat spontaneously but, for various reasons, do not consume enough nutrients. For instance, dietary supplements are often utilised in nursing homes as well as hospitals to treat elderly patients suffering from or at risk of protein-calorie malnutrition due to illness or age.

Dietary supplements are also useful for patients who do not absorb adequate nutrition from a routine diet. For instance, individuals who have high energy needs, fluid restriction or fat malabsorption can benefit from the use of dietary supplements. For persons who have an isolated deficiency, supplements can increase the total quantity of carbohydrate, fat or protein consumed.

The use of complete oral formulas as dietary supplements for patients who are deficient in their intake of protein, carbohydrate and/or fat is frequently a temporary measure. The temporary nature of such dietary supplements often stems from the patient's resistance to continually ingest such formulas over time as opposed to the continued need for the supplements. The great majority of medical food supplements are liquids intended for consumption at room or refrigerated temperatures.

Variety with these products currently means different flavours of the same form. However, research on sensory-specific satiety shows that consumption of foods and nutritional supplements with an almost identical hedonic profile, regardless of flavour differentiation, results in a sense of satiety and boredom. As a result, a progressive drop in compliance of the use of the supplements arises. Moreover, some carry-over satiety that affects the intake of other food as well as a lower caloric intake/day arises.

Moreover, in addition to taste variety, the nutritional supplement must also contain a nutritionally complete profile of needed nutrients to provide the required nutritional support. An assortment of meal supplements and snacks exist on the market, such as many weight loss and sports supplements. However, these supplements tend to stress low fat or high protein without providing nutritional balance and completeness in the form of the full component of vitamins and minerals. Patients consuming such products fail to obtain the required nutrients needed for adequate nutritional support.

Therefore, a need exists for a complete nutritional supplement in a new form or flavour offered to assist in providing nutrition in a variety of forms and flavours.

Accordingly, in one aspect, this invention provides a nutritional composition that, when reconstituted with hot water, provides a complete, nutritionally balanced coffee drink, the nutritional composition being in the form of an agglomerated, dissolvable powder comprising: soluble coffee powder; 16% to 30% of calories of a protein component; 40% to 60% of calories of a carbohydrate component; and 15% to 33% of calories of a lipid component.

The nutritional composition, when reconstituted with hot water, provides a nutritionally complete, balanced coffee drink which may be used to provide caloric and protein support to a patient in need of nutritional support. Hence the composition provides a nutritional supplement in the form of a traditional coffee drink, which looks and tastes like black coffee, but which contains complete and balanced nutritional components. Further, the composition may be used to stimulate appetite and prevent taste fatigue by contributing to the variety of suitable nutritional forms. The stimulation of the appetite in turn encourages proper nutrition.

The nutritional composition and coffee drink are especially useful as an enterally administered component in a program of nutritional care and management which utilises a number of carefully designed nutritional products in various forms. For example products in shake, soup, fruit drink, snack bar and coffee drink forms. Therefore the products may be mixed and matched over a period of nutritional care to provide more attractive and, therefore, more effective nutritional support to a patient, particularly those in extended care situations.

Conveniently, the soluble coffee powder may comprises decaffeinated coffee.

Preferably, the protein component comprises at least 25% of calories of the composition. Further, the protein component comprises one or more of whey, milk protein, vegetable protein, peptide, di-peptide, and oligo-peptide.

The protein component preferably has a concentration of high quality nutritional protein of from 78% to 92% of calories.

The carbohydrate component preferably comprises maltodextrin. Further, the carbohydrate component preferably comprises 55% of calories.

Preferably the composition has a bulk density of from 250 to 290 kg/m³. The composition preferably has a particle size capable of passing through a sieve having a mesh size of from 5 to 12 openings per cm.

The composition preferably comprises at least 1/2000 of US RDA of vitamins and minerals per calorie of the composition.

In another aspect, this invention provides a process for the production of a nutritional composition that, when reconstituted with hot water, provides a complete, nutritionally balanced coffee drink, the process comprising:
dry blending a powdered protein component and a powdered carbohydrate component to form a first mixture;
spraying an aqueous salt solution onto the first mixture while dry blending and agglomerating to form a second mixture;
spraying an atomised oil onto the second mixture to form a third mixture;
dry blending the third mixture with coffee powder to form a fourth mixture; and
spraying an aqueous carbohydrate solution onto the fourth mixture while dry blending and agglomerating to provide the nutritional composition.

Preferably the process further comprises sieving the nutritional composition through a sieve having a mesh size of from 5 to 12 openings per cm.

A powdered vitamin premix and a powdered trace elements premix are preferably dry blended with the powdered protein component and the powdered carbohydrate component to form the first mixture.

The aqueous salt solution may comprise potassium citrate, sodium biphosphate and choline bitartrate. The aqueous carbohydrate solution may comprise a maltodextrin solution.

The invention further comprises a nutritional composition produced by the process defined above.

In another aspect, this invention provides a complete, nutritionally balanced coffee drink comprising an aqueous solution of:
soluble coffee solids;
16% to 30% of calories of a protein component;
40% to 60% of calories of a carbohydrate component; and
15% to 33% of calories of a lipid component,
the coffee drink having the taste and appearance of black coffee.

In a yet further aspect, this invention provides a method for providing nutrition to a patient in need of nutritional support, the method comprising enterally administering to the patient a balanced, nutritionally complete coffee drink as defined above.

In a further aspect, this invention provides a method for providing additional calories and protein to an elderly patient, the method comprising enterally administering to the elderly patient a dietary supplement in the form of a coffee drink as defined above.

Embodiments of the invention are now described, by way of example only.

Nutritional support of hospitalised as well as non-hospitalised patients requires prevention, recognition and treatment of nutritional depletion that may occur with illness. The goals of nutritional support include stabilising metabolic state, maintaining body mass, and/or facilitating growth in the presence of disease and gastrointestinal dysfunction.

Ensuring proper nutritional support depends on obtaining and maintaining patient compliance. To this end, a never-ending market demand for oral supplements is providing taste variety. Variety stimulates the appetite of the patient and thereby prevents taste fatigue. Stimulation of the appetite in turn encourages proper nutrition. Therefore, to facilitate nutritional support of hospitalised as well as non-hospitalised patients, a nutritional composition is provided which increases variety.

The composition comprises soluble coffee powder, a protein component, a carbohydrate component and a lipid component.

Any suitable protein source may be used as the protein component. Preferably the protein source is able to exhibit good heat stability, good solubility in water and high clarity in aqueous solution so that the coffee colour appears black. Suitable protein sources are whey protein, milk protein, vegetable protein such as soy and pea proteins and mono-, di- and oligo-peptides. Preferably the protein source is a whey protein or caseinate; for example whey protein isolate or sodium caseinate.

The protein component preferably provides approximately 16% to 30% of the calories of the composition; for example approximately 25% to 30% of the calories of the composition. Preferably the protein component comprises 78% to 92%, of protein calories, of a protein of high nutritional quality; for example casein or whey proteins.

Any suitable carbohydrate source may be used as the protein component; for example maltodextrin, corn syrup solids and sucrose. The carbohydrate component preferably provide approximately 40% to 60% of the calories of the composition; more preferably approximately 50% to about 55% of the calories of the composition. For example, the carbohydrate component comprises approximately 55% of the calories of the composition.

In addition to simple sugars, the carbohydrate source may include a source of dietary fibre. Numerous types of dietary fibre are available. Dietary fibre passes through the small intestine undigested by enzymes and represents a kind of natural and necessary laxative. Suitable sources of dietary fibre, among others, include soy, oat, and gum arabic. Up to approximately 14 g/L total fibre may be added to the composition.

Any suitable lipid source may be used as the lipid component. Preferably however the lipid source includes of long chain triglycerides (LCT); for example a mixture of long chain triglycerides. Suitable sources of long chain triglycerides are olive oil, corn oil, canola oil, palm kernel oil, sunflower oil, peanut oil, soy lecithin and residual milk fat. The lipid profile containing such long chain triglycerides is designed to have a polyunsaturated fatty acid omega-6 (n-6) to omega-3 (n-3) ratio of approximately 3:1 to 6:1. The proposed ratio of n-6:n-3 is designed to prevent suppression of the immune system caused by excessive n-6 fatty acids. Preferably, the omega-6 to omega-3 ratio is approximately 4:1.

While the lipid source preferably contains only long-chain triglycerides, the fat source may also contain medium chain triglycerides; for example the lipid source may comprise up to approximately 75%, of calories, of medium chain triglycerides. Medium chain triglycerides are easily absorbed and metabolised in the patient's body.

In addition to proper amounts of protein, lipid and carbohydrates, most patients receiving dietary supplements have elevated requirements for certain vitamins, minerals and trace elements. Therefore the composition preferably includes a suitable vitamin and mineral profile; for example at least 1/2000 of the U.S. RDA of vitamins and minerals per calorie of the composition.

The composition may also include a source of beta-carotene. Beta-carotene meets a portion of the required vitamin A, thereby meeting micronutrient requirements in a small caloric volume. It is also an important nutrient with antioxidant properties. The composition preferably includes approximately 0.5 to 1.5 mg/1000 calories of beta-carotene. Preferably the beta-carotene is present in an amount of approximately 1.0 mg/1000 calories.

By way of example, and not limitation, an example of a suitable composition is as follows:

The composition includes the following ingredients:
soluble coffee solids;
protein (for example caseinate);
carbohydrate (for example maltodextrin, corn syrup solids, or sucrose, and mixtures thereof);
fat (for example canola oil, corn oil, or soy lecithin and mixtures thereof); and
vitamins and minerals (for example vitamin A, beta-carotene, vitamin D, vitamin E, vitamin K, vitamin C, thiamine (B₁), riboflavin (B₂), niacin, vitamin B₆, folic acid, pantothenic acid, vitamin B₁₂, biotin, choline, taurine, calcium, phosphorus, magnesium, zinc, iron, copper, manganese, iodine, sodium, potassium, chloride, chromium, molybdenum or selenium, and mixtures thereof).

The composition is preferably lactose-free, gluten-free, low in sodium and low in cholesterol. Further the composition preferably has a caloric density of approximately 0.75 to 2.0 kcal/ml. The osmolality of the composition preferably ranges from approximately 450 to 750 mOsm.

The composition may provided as a powder or in the form of a ready-to-use oral supplement.

The composition may be prepared by mixing the protein and carbohydrate components together, alone or in combination, with any vitamins, trace elements and salts that are desired. The oil or oils forming the lipid component are then sprayed onto the mixture. For best appearance of the ultimate beverage, care should be taken to atomise the oil to a sufficiently small particle size so that, after reconstitution with hot water, no sign of a milky oil appears. Control of the particle size of the oil may generally be provided by controlling the nozzle diameter, delivery pressure and spraying time.

The soluble coffee powder then may be mixed in. Any fillers or dissolution agents may be added at this time. The mixture may then be agglomerated; for example by dry mixing. The agglomerated composition may then be sieved through a sieve having a mesh size of from 5 to 12 openings per cm.

The composition is not disease specific and therefore may be utilised to provide adequate nutrition to a multitude of long-term care patients, such as patients suffering from AIDS, malabsorbing conditions, or other chronic diseases. Moreover, the composition is suitable for providing nutrition to elderly patients suffering from or at risk of protein-calorie malnutrition due to illness or age.

By way of example, and not limitation, examples of suitable nutritional product forms and corresponding nutrient profiles that may be used pursuant to the present invention will now be given.

### Example 1 - Reconstitutable Coffee Composition

A composition is produced which, when reconstituted with hot water, provides a coffee drink which looks and tastes like black coffee. A first dry mix is prepared by mixing 159.5 kg of carbohydrate (Maltrin M-180), 79.7 kg of protein (Whey protein isolate), 2.4 kg of calcium carbonate, 0.9 kg of magnesium carbonate, about 1 kg of a vitamin premix and about 0.5 kg of a trace mineral element premix in an IFP agglomerator obtained from Glatt AG. The temperature of the components is maintained at about 34°C to about 37°C during mixing. Mixing is continued for about 2 minutes.

A salt solution is prepared by dissolving about 3 kg of potassium citrate, about 1.9 kg of sodium biphosphate and about 0.5 kg of choline bitartrate to about 35.3 kg water. The salt solution is sprayed onto the dry mix through a 1.8 mm nozzle under a pressure of about 350 kPa over a period of about 13 minutes. Mixing is continued during spraying to cause agglomeration. The temperature of components is maintained at about 36°C to about 40°C during spraying.

About 24.5 kg of a sunflower oil (obtainable from SVO Specialty Products Inc. of Eastlake, Ohio 44095, USA under the trade name Trisun Oil R 80) is sprayed onto the agglomerated mixture at a pressure of about 275 kPa over a period of about 9 minutes. The diameter of the nozzle aperture is about 1.8 mm. The temperature of the components is from about 55° C to about 60°C during spraying.

About 25.1 kg of a commercial, decaffeinated soluble coffee powder (Nescafé® Decaffeinated) is then added and the mixture of ingredients is mixed for about 1 minute. The temperature of the mixture is maintained at about 55°C to about 60°C.

An aqueous maltodextrin solution is prepared by adding about 3 kg of maltodextrin (Maltrin M-180) to about 15.1 kg of water at 65°C. The solution is then sprayed onto the mixture through a 1.8 mm nozzle under a pressure of about 350 kPa over a period of about 6 to 8 minutes. The temperature of the mixture is maintained at about 55°C to about 58°C. An agglomerated mixture is obtained.

The agglomerated mixture is sieved through a sieve having a mesh size of 6 openings per cm and stored in 25 kg bags. The agglomerated mixture has a bulk density of 250 to 290 kg/m³ and a tapped density of 360 to 400 kg/m³.

### Example 2

Two scoops of the agglomerated mixture of example 1 (about 34 g) are dissolved in about 180 ml to about 240 ml of hot water. The mixture dissolved almost instantly to provide a coffee drink in the form of clear solution looking and tasting like black coffee and which contained nutritionally therapeutic amounts of calories, protein, carbohydrate and lipid.

Further, the coffee drink provides therapeutically effective amounts of high quality protein and calories in a nutritionally balanced and complete coffee beverage. The composition is especially well suited for use by the elderly in general, whether in institutions or at home, who frequently are undernourished with respect to their protein and caloric intakes. The elderly frequently consume coffee but most coffee drinks are of insufficient nutritional value.

## Claims

1. A nutritional composition that, when reconstituted with hot water, provides a complete, nutritionally balanced coffee drink, the nutritional composition being in the form of an agglomerated, soluble powder comprising:
soluble coffee powder;
a protein component providing 16% to 30% of calories;
a carbohydrate component providing 40% to 60% of calories; and
a lipid component providing 15% to 33% of calories.

2. A composition according to claim 1 in which the protein component provides 25% to 30% of calories.

3. A composition according to claim 1 or claim 2 in which the protein component includes high quality nutritional protein providing 78% to 92% of calories of the protein component.

4. A composition according to any of claims 1 to 3 in which the protein component is one or more of whey, milk protein, vegetable protein, peptide, di-peptide, and oligo-peptide.

5. A composition according to any of claims 1 to 4 in which the carbohydrate component comprises maltodextrin.

6. A composition according to any of claims 1 to 5 which has a bulk density of from 250 to 290 kg/m³.

7. A composition according to any of claims 1 to 6 which has a particle size capable of passing through a sieve having a mesh size of from 5 to 12 openings per cm.

8. A process for the production of a nutritional composition according to claim 1, the process comprising:
dry blending a powdered protein component and a powdered carbohydrate component to form a first mixture;
spraying an aqueous salt solution onto the first mixture while dry blending and agglomerating to form a second mixture;
spraying an atomised oil onto the second mixture to form a third mixture;
dry blending the third mixture with coffee powder to form a fourth mixture; and
spraying an aqueous carbohydrate solution onto the fourth mixture while dry blending and agglomerating to provide the nutritional composition.

9. The use of soluble coffee solids in the preparation of a powdered nutritional composition that, when reconstituted with hot water, provides a complete, nutritionally balanced coffee drink comprising an aqueous solution of: soluble coffee solids; 16% to 30% of calories of a protein component; 40% to 60% of calories of a carbohydrate component; and 15% to 33% of calories of a lipid component; for providing nutrition to a patient in need of nutritional support.

10. The use of soluble coffee solids in the preparation of a powdered nutritional composition that, when reconstituted with hot water, provides a complete, nutritionally balanced coffee drink comprising an aqueous solution of: soluble coffee solids; 16% to 30% of calories of a protein component; 40% to 60% of calories of a carbohydrate component; and 15% to 33% of calories of a lipid component; for providing additional calories and protein to an elderly patient.

## Patentansprüche

1. Nährzusammensetzung, die, wenn sie mit heißem Wasser rekonstituiert ist, ein vollständiges, ernährungsphysiologisch ausgewogenes Kaffeegetränk liefert, wobei die Nährzusammensetzung in Form eines agglomerierten löslichen Pulvers vorliegt, das umfaßt:
lösliches Kaffeepulver;
eine Proteinkomponente, die 16% bis 30% der Kalorien liefert;
eine Kohlenhydratkomponente, die 40 bis 60% der Kalorien liefert; und
eine Lipid-Komponente, die 15 bis 33% der Kalorien liefert.

2. Zusammensetzung nach Anspruch 1, bei der die Proteinkomponente 25 bis 30% der Kalorien liefert.

3. Zusammensetzung nach Anspruch 1 oder Anspruch 2, bei der die Proteinkomponente ein Nahrungsprotein hoher Qualität einschließt, das 78 bis 92% der Kalorien der Proteinkomponente liefert.

4. Zusammensetzung nach irgendeinem der Ansprüche 1 bis 3, bei der die Proteinkomponente eines oder mehrere von Molke, Milchprotein, Pflanzenprotein, Peptid, Dipeptid oder Oligo-Peptid ist.

5. Zusammensetzung nach irgendeinem der Ansprüche 1 bis 4, bei der die Kohlenhydratkomponente Maltodextrin umfaßt.

6. Zusammensetzung nach irgendeinem der Ansprüche 1 bis 5, die eine Rohdichte von 250 bis 290 kg/m³ aufweist.

7. Zusammensetzung nach irgendeinem der Ansprüche 1 bis 6, die eine solche Teilchengröße aufweist, dass sie in der Lage ist, ein Sieb mit einer Maschengröße von 5 bis 12 Öffnungen pro cm zu passieren.

8. Verfahren zur Herstellung einer Nährzusammensetzung nach Anspruch 1, wobei das Verfahren umfaßt:
Trockenmischen einer pulverförmigen Proteinkomponente und einer pulverförmigen Kohlenhydratkomponente, um eine erste Mischung zu bilden;
Aufsprühen einer wäßrigen Salzlösung auf die erste Mischung, während man diese trocken mischt und agglomeriert, um eine zweite Mischung zu bilden;
Aufsprühen eines atomisierten Öls auf die zweite Mischung, um eine dritte Mischung zu bilden;
Trockenmischen der dritten Mischung mit Kaffeepulver, um eine vierte Mischung zu bilden; und
Aufsprühen einer wäßrigen Kohlenhydratlösung auf die vierte Mischung, während man diese trocken mischt und agglomeriert, um die Nährzusammensetzung zu erhalten.

9. Verwendung von löslichen Kaffeefeststoffen bei der Herstellung einer pulverförmigen Nährzusammensetzung, die, wenn sie mit heißem Wasser rekonstituiert ist, ein vollständiges, ernährungsphysiologisch ausgewogenes Kaffeegetränk liefert, das eine wäßrige Lösung von:
löslichen Kaffeefeststoffen; 16% bis 30% der Kalorien einer Proteinkomponente; 40 bis 60% der Kalorien einer Kohlenhydratkomponente; und 15 bis 33% der Kalorien einer Lipidkomponente aufweist, zur Nahrungszufuhr an einen Patienten, der ernährungsmäßig einer Unterstützung bedarf.

10. Verwendung von löslichen Kaffeefeststoffen bei der Herstellung einer pulverförmigen Nährzusammensetzung, die, wenn sie mit heißem Wasser rekonstituiert ist, ein vollständiges, ernährungsphysiologisch ausgewogenes Kaffeegetränk liefert, das eine wäßrige Lösung von:
löslichen Kaffeefeststoffen; 16 bis 30% der Kalorien einer Proteinkomponente; 40 bis 60% der Kalorien einer Kohlenhydratkomponente; und 15 bis 33% der Kalorien einer Lipidkomponente aufweist, um einem älteren Patienten zusätzliche Kalorien und Protein zuzuführen.

## Revendications

1. Composition nutritionnelle qui, une fois reconstituée avec de l'eau chaude, donne une boisson au café complète, nutritionnellement équilibrée, la composition nutritionnelle étant sous forme d'une poudre soluble agglomérée comprenant :
une poudre de café soluble ;
un constituant protéique fournissant 16 % à 30 % des calories ;
un constituant glucidique fournissant 40 % à 60 % des calories ; et
un constituant lipidique fournissant 15 % à 33 % des calories.

2. Composition suivant la revendication 1, dans laquelle le constituant protéique fournit 25 % à 30 % des calories.

3. Composition suivant la revendication 1 ou la revendication 2, dans laquelle le constituant protéique comprend une protéine nutritionnelle de grande qualité fournissant 78 % à 92 % des calories du constituant protéique.

4. Composition suivant l'une quelconque des revendications 1 à 3, dans lesquelles le constituant protéique est un ou plusieurs des ingrédients consistant en le petit-lait, une protéine laitière, une protéine végétale, un peptide, un dipeptide et un oligopeptide.

5. Composition suivant l'une quelconque des revendications 1 à 4, dans laquelle le constituant glucidique comprend la maltodextrine.

6. Composition suivant l'une quelconque des revendications 1 à 5, qui a une masse volumique de 250 à 290 kg/m³.

7. Composition suivant l'une quelconque des revendications 1 à 6, qui a un diamètre de particules lui permettant de passer à travers un tamis ayant des dimensions des mailles correspondant à 5 à 12 ouvertures par cm.

8. Procédé pour la production d'une composition nutritionnelle suivant la revendication 1, procédé comprenant les étapes consistant :
à mélanger à sec un constituant protéique en poudre et un constituant glucidique en poudre pour former un premier mélange ;
à pulvériser une solution aqueuse de sels sur le premier mélange tout en effectuant un mélange à sec et une agglomération pour former un deuxième mélange ;
à pulvériser une huile atomisée sur le deuxième mélange pour former un troisième mélange ;
à mélanger à sec le troisième mélange à de la poudre de café pour former un quatrième mélange ; et
à pulvériser une solution aqueuse de glucide sur la quatrième mélange, tout en effectuant un mélange à sec et une agglomération pour produire la composition nutritionnelle.

9. Utilisation de matière sèche de café soluble dans la préparation d'une composition nutritionnelle en poudre qui, une fois reconstituée avec de l'eau chaude, donne une boisson au café complète nutritionnellement équilibrée, comprenant une solution aqueuse : de matière sèche de café soluble ; de 16 % à 30 %, des calories, d'un constituant protéique ; de 40 % à 60 %, des calories, d'un constituant glucidique ; et de 15 % à 33 %, des calories, d'un constituant lipidique ; pour assurer la nutrition d'un patient nécessitant un apport nutritionnel.

10. Utilisation de matière sèche de café soluble dans la préparation d'une composition nutritionnelle en poudre qui, une fois reconstituée avec de l'eau chaude, donne une boisson au café complète nutritionnellement équilibrée, comprenant une solution aqueuse : de matière sèche de café soluble ; de 16 % à 30 %, des calories, d'un constituant protéique ; de 40 % à 60 %, des calories, d'un constituant glucidique ; et de 15 % à 33 %, des calories, d'un constituant lipidique ; pour fournir des calories et protéines supplémentaires à un patient âgé.
